# EUROPEAN PATENT SPECIFICATION

(11) **EP 2 702 964 B1**
(45) Date of publication and mention of the grant of the patent: **19.07.2017**
(21) Application number: 12776498.3
(22) Date of filing: 23.04.2012
(51) Int. Cl.: A61F 2/07, A61F 2/915, A61F 2/848

(54) **STENT**
STENT
STENT

(30) Priority: 27.04.2011 JP 2011098892
(43) Date of publication of application: 05.03.2014
(73) Proprietor: Piolax Medical Devices, Inc., Yokohama-shi Kanagawa 240-0023 (JP)
(72) Inventor: TOYOKAWA, Yoshihide, Yokohama-shi, Kanagawa 240-0023 (JP)
(74) Representative: Grünecker Patent- und Rechtsanwälte PartG mbB
(86) International application number: PCT/JP2012/060825
(87) International publication number: WO 2012/147675

(56) References cited:
- WO-A2-2009/094188
- JP-A- 2000 167 063
- JP-A- 2011 509 805
- US-A1- 2003 176 911
- US-A1- 2010 076 547

## Description

### Technical Field

The present invention relates to a stent which is placed in a tubular organ such as a blood vessel, a ureter, a bile duct or a trachea to thereby prevent stenosis or occlusion of the tubular organ, rupture of an aneurysm etc.

### Background Art

Recently, a method of treatment using a stent has been provided. In such a method, the stent is placed in a stenosed portion or an occluded portion in a tubular organ of a human body such as a blood vessel, a ureter, a bile duct or a trachea so as to dilate the stenosed portion or the occluded portion, or the stent is placed in a portion where an aneurysm has occurred, so as to prevent the aneurysm from rupturing.

This kind of stent is received, for example, inside a catheter, a sheath, or the like, in the state in which the diameter of the stent is reduced. After the stent is carried to a predetermined position of the tubular organ in that state, the stent is pushed out by a pusher etc. so that the stent can be placed in close contact with an inner wall of the tubular organ.

After the stent is placed thus in the tubular organ, if the lumen of the tubular organ is occluded by a body fluid such as bile as time goes on, the stent should be taken out from the tubular organ, and a new stent should be placed. In addition, after the treatment has been completed, the stent should be taken out from the tubular organ.

JP 2009178545 A discloses a biodegradable double-structure stent which can be taken out from a tubular organ after having been placed. The biodegradable double-structure stent includes a body stent and a separately-provided biodegradable stent. The body stent is formed into a hollow cylindrical body by knitting alloy wires crosswise to form a large number of rhombic space portion. The body stent is inserted inside an internal organ to widen a duct. The biodegradable stent is formed into a hollow cylindrical body by knitting biodegradable polymer wires made of a biodegradable polymer crosswise to form a large number of rhombic space. The biodegradable stent has a bulging portion protruding into a bulging shape in the center portion thereof. The biodegradable stent is located outside the body stent such that one of opposite ends of the biodegradable stent is connected to the body stent. The bulging portion annularly bulges over the whole circumference of the biodegradable stent.

WO 2009/094188 A2 discloses a stent for prosthetic heart valves with the features of the preamble of claim 1. The stent includes a generally tubular body portion made of a metal wire and comprising fixation taps which are configured as bulging wire areas and minimize or prevent migration of the stent.

When the bulging portion of the biodegradable stent is locked on an inner wall of a tubular organ, the double-structure stent is placed in a predetermined position of the tubular organ. Since the biodegradable stent made of the biodegradable polymer is dissolved gradually by a body fluid when the biodegradable stent is dissolved and gone, the body stent located inside the biodegradable stent can be taken out from the tubular organ.

### Citation List

### Patent Literature

**Patent Literature 1** JP-2009-178545-A

### Summary of Invention

### Technical Problem

The double-structure stent in Patent Literature 1 can indwell inside the tubular organ due to the use of the biodegradable stent made of the biodegradable polymer. Accordingly, when the duration of the indwelling is long, the biodegradable stent may be dissolved, with the result that the double-structure stent may move from the predetermined position of the tubular organ unsuitably for the long-term indwelling.

In addition, the annularly bulging portion provided over the whole circumference of the biodegradable stent is locked on the inner wall of the tubular organ. For this reason, it will be difficult to take out the double-structure stent from the tubular organ in a short period of time before the biodegradable stent is completely dissolved, due to the excessive large force with which the biodegradable stent is locked on the tubular organ.

Accordingly, an object of the invention is to provide a stent which can be placed accurately in a predetermined position of a tubular organ without displacement for a long term and which can be taken out smoothly from the inside of the tubular organ at a desired time.

### Solution to Problem

Insofar as the terms "invention" and/or "embodiment" are used in the following, and/or features are presented as being optional, this should be interpreted in such a way that the only protection sought is that of the invention as claimed. In order to achieve the foregoing object, the invention provides a stent with the features of claim 1 which is placed in a tubular organ, including: a metal stent body; a resin cover member which covers at least a part of an outer circumference of the stent body; and bulging portions which are provided in the part of the outer circumference of the stent body covered with the cover member and which make close contact with an inner wall of the tubular organ so as to restrain the stent from moving, wherein each of the bulging portions has a gradually-bulged shape which extends with a predetermined width in a circumferential direction and with a predetermined length in an axial direction so as to bulge gradually and then decline gradually.

In the stent, preferably, the bulging portions are disposed in plural places at predetermined intervals along the circumferential direction of the stent body and in plural places at predetermined intervals also along the axial direction of the stent body so as not to overlap one another in axial view from an end face of the stent body.

In the stent, preferably, each of the bulging portions is formed so that the length of the bulging portion along the axial direction of the stent body is larger than the width of the bulging portion along the circumferential direction of the stent body.

In the stent, preferably, a space is formed inside each of the bulging portions.

In the stent, preferably, inclination of each of the bulging portions along the axial direction of the stent body is formed so that inclination of a slope corresponding to a direction of taking out the stent with respect to a vertex portion is gentler than inclination of an opposite slope.

### Advantageous Effects of Invention

According to the invention, when the stent is placed inside a tubular organ through a catheter, a sheath, or the like, the bulging portions make close contact with an inner wall of the tubular organ. Therefore, the stent can be restrained from being displaced so as to be placed accurately in a predetermined position of the tubular organ. Since each of the bulging portions is formed into a shape protruding smoothly so as to bulge gradually and then decline gradually, even when the stent needs to be taken out, the stent can be extracted relatively smoothly without damaging the tubular organ.

### Brief Description of Drawings

[Fig. 1] An exploded perspective view showing an embodiment of a stent according to the invention.
[Fig. 2] A perspective view of the stent.
[Fig. 3] Views showing a stent body forming the stent, in which (a) is a development view of the stent body and (b) is a development view of a stent body in another example.
[Fig. 4] (a) is a sectional view taken along the line of arrows A-A in Fig. 2 and (b) is a sectional view taken along the line of arrows B-B in Fig. 2.
[Fig. 5] (a) is a main part plan view of the stent according to the invention, and (b) is a main part side view of the stent.
[Fig. 6] A sectional view in which the stent according to the invention is cut along an axial direction.
[Fig. 7] An explanatory view in the case where the stent is placed in a tubular organ.
[Fig. 8] Views showing layout examples of bulging portions in the stent, in which (a) is a sectional view in the state in which the bulging portions are disposed to be opposite to each other in a circumferential direction of the stent body, and (b) is a sectional view in the state in which four bulging portions are disposed at equal intervals in a circumferential direction of the stent body.
[Fig. 9] A perspective view showing another embodiment of the stent according to the invention.
[Fig. 10] Explanatory views showing other shapes of the bulging portions in the stent.

### Description of Embodiments

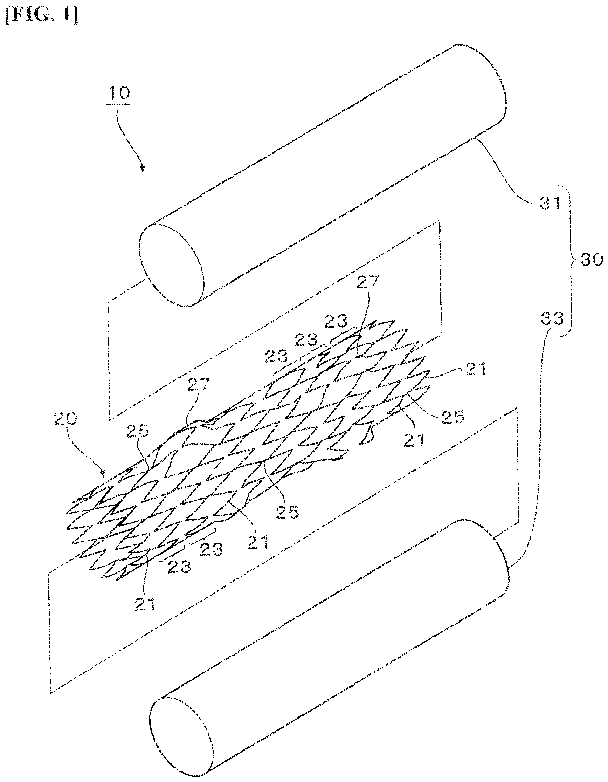

An embodiment of a stent according to the invention will be described below with reference to Figs. 1 to 8.

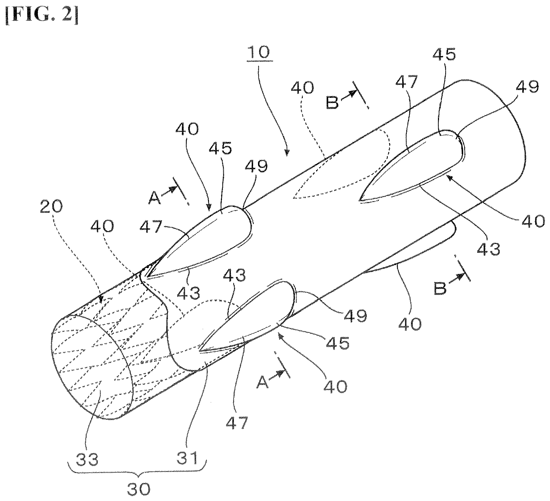

As shown in Figs. 1 and 2, a stent 10 in this embodiment has a metal stent body 20, a resin cover member 30 which covers at least a part of an outer circumference of the stent body 20, and bulging portions 40 which are provided in the part of the outer circumference of the stent body 20 covered with the cover member 30 and which make close contact with an inner wall of a tubular organ so as to restrain the stent 10 from moving.

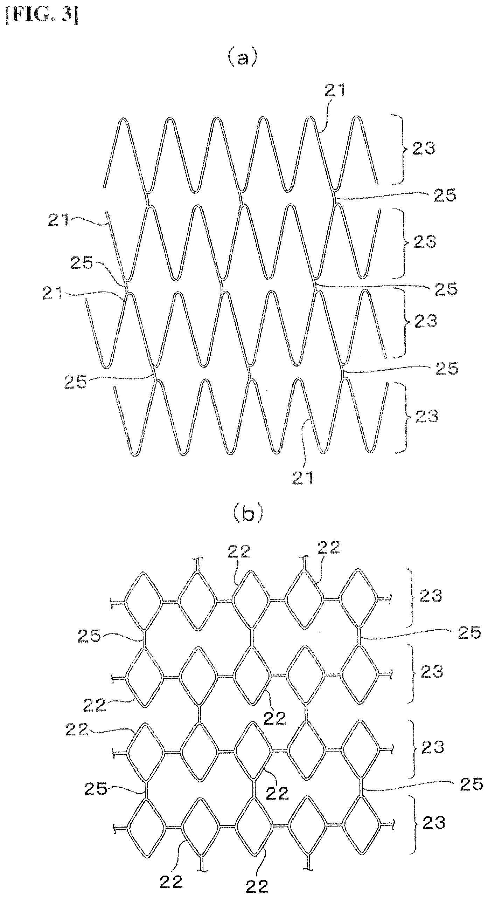

In the embodiment, the stent body 20 is formed by processing a metal cylinder. As shown in Fig. 1 and Fig. 3(a), a metal cylinder is processed by laser processing, etching or the like to thereby form circumferential units 23 each having an annularly-connected zigzag shape. Bent portions of the circumferential units 23 are connected to one another through connection portions 25 such that the plural circumferential units 23 are connected to one another axially, thereby forming the sent body 20 having a cylinder-like shape.

Alternatively, as shown in Fig. 3(b), the cylinder-shaped stent body 20 may be formed by processing a metal cylinder such that plural circumferential units 23 each formed of plural frame-like bodies 22 are connected to one another axially through plural connection portions 25.

A cylinder-shaped stent body 20 may be formed by knitting metal wires, for example, by weaving, braiding or entwining metal wires.

The material of the stent body 20 is not limited particularly. For example, stainless steel, Ta, Ti, Pt, Au, W etc., a shape memory alloy such as an Ni-Ti-based alloy, a Co-Cr-based alloy, a Co-Cr-Ni-based alloy, a Cu-Zn-X (X=Al, Fe, etc.) alloy or an Ni-Ti-X (X=Fe, Cu, V, Co, etc.) alloy, or the like, may be used preferably as the material of the stent body 20.

When the shape memory alloy is used, shape memory treatment is applied to the metal cylinder which has been expanded in diameter. In this manner, it is possible to obtain a self-expandable type stent body 20 which can be expanded in diameter at a temperature not lower than a transformation point. The stent body 20 may be of a balloon dilatable type which can be dilated in diameter by a balloon catheter.

As shown in Figs. 1, 2, 4 and 6, the resin cover member 30 in the embodiment includes an outer cover 31 disposed on an outer circumference of the stent body 20 and an inner cover 33 which is disposed on an inner circumference of the stent body 20. Each of the covers 31 and 33 has a cylindrical shape formed over the whole length of the stent body 20 so that the inner and outer circumferences of the stent body 20 are wholly covered with these covers 31 and 33.

As materials of each cover 31, 33, for example, polyurethane, silicone, natural rubber, nylonelastomer, polyether block amide, polyethylene, polyvinyl chloride, vinyl acetate, and further fluorine-based resins such as polytetrafluoroethylene (PTFE), a perfluoroalkoxy resin (PFA), a fluorinated ethylene propylene copolymer (FEP) and an ethylene tetrafluoroethylene copolymer (ETFE) may be preferably used. The outer circumference of each cover 31, 33 may be further coated with polybutadiene, styrene-based elastomer, silicone, etc., so that polyurethane, nylonelastomer etc. which are hydrolyzed easily can be protected.

Protruding portions 27 are provided protrusively in the outer circumference of the stent body 20 (see Fig. 1). When the stent body 20 is covered with the resin cover member 30, bulging portions 40 are provided in the portion of the outer circumference of the stent body 20 covered with the outer cover 31. Each of the bulging portions 40 extends with a predetermined width in the circumferential direction of the stent body 20 and with a predetermined length in the axial direction of the stent body 20. The bulging portion 40 is formed into a shape protruding smoothly so as to bulge gradually and then decline gradually. That is, since the resin cover member 30 is pushed out from inside by the protruding portions 27, the outer cover 31 of the cover member 30 covering the outer circumference of the stent body 20 is also formed into a shape bulging in accordance with the shape of each protruding portion 27. In the following description, the protruding portions 27 of the stent body 20 and the bulging portions of the outer cover 31 covering the surface of the stent body 20 are collectively referred to as bulging portions 40.

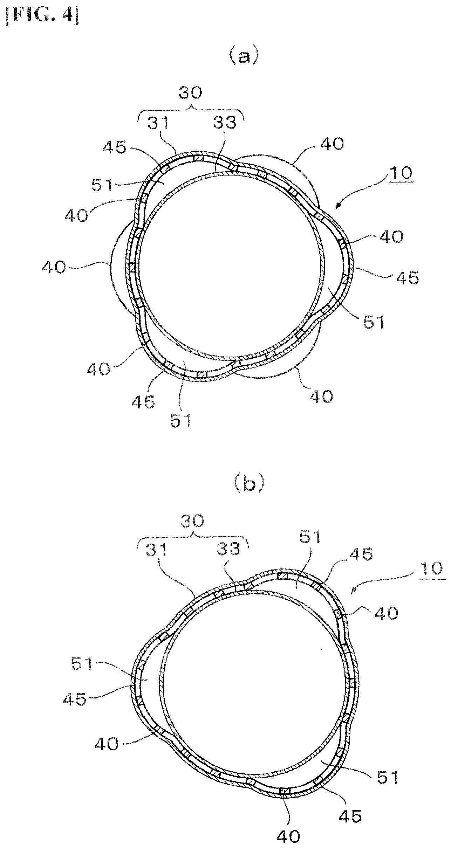

In the embodiment, the bulging portions 40 are disposed in plural places at predetermined intervals in accordance with the circumferential direction of the stent body 20 and also disposed in plural places at predetermined intervals in the axial direction of the stent body 20. The bulging portions 40 are disposed so as not to overlap one another in axial view from one end face of the stent body 20. Specifically, as shown in Fig. 2 and Figs. 4(a) and 4(b), three bulging portions 40 are disposed at circumferentially equal intervals in places closer to one axial end of the stent body 20, while three bulging portions 40 are disposed at circumferentially equal intervals in places closer to the other axial end of the stent body 20 so as not to overlap the bulging portions 40 provided in the places closer to the one end.

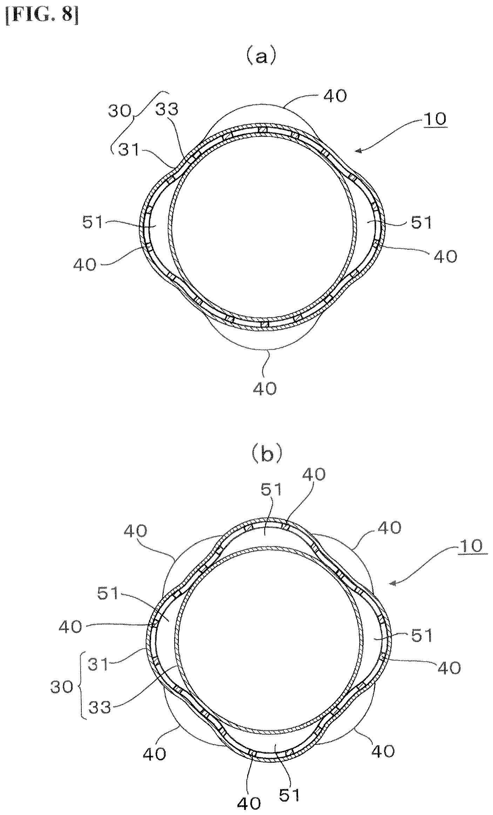

As shown in Fig. 8(a), bulging portions 40 and 40 are provided in opposite places in the circumferential direction of the stent body 20. Four bulging portions 40 may be provided in equal intervals in the circumferential direction of the stent body 20 as shown in Fig. 8(b), or more bulging portions 40 may be disposed. The bulging portions 40 may be provided in plural places at predetermined intervals in the axial direction of the stent body 20 so as not to overlap one another when the stent body 20 is viewed from an axial end face thereof, as shown in Figs. 8(a) and 8(b).

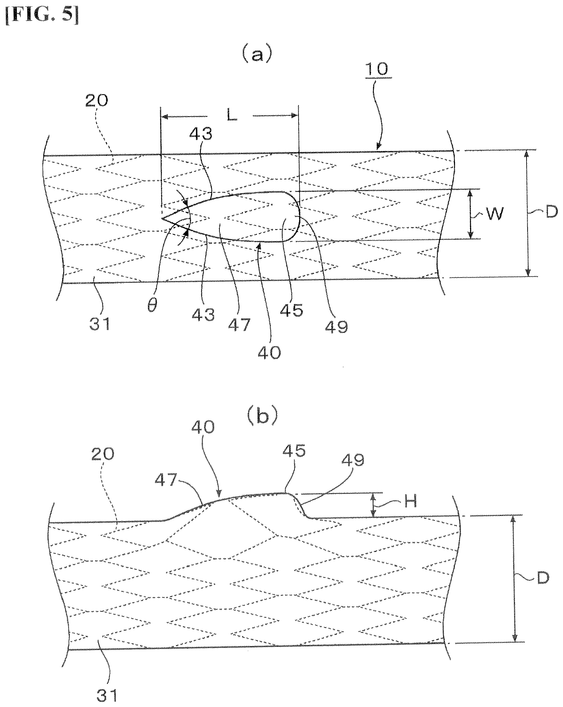

As shown in Fig. 2 and Fig. 5(a), each of the bulging portions 40 in the embodiment has a tapered shape with sharpness on the axially distal end side and has a water-drop-like shape which is expanded in diameter so that the width between opposite edges 43 and 43 increases gradually toward the axially proximal end side while the edges 43 and 43 draw curves. The bulging portion 40 is disposed so that the distal end side forming the tapered shape indicates an extraction direction in which the stent 10 will be extracted.

As shown in Fig. 2, Fig. 5(b) and Fig. 6, the portion of the bulging portion 40 along the axial direction of the stent body 20 has a shape which bulges gradually higher in level from the distal end side toward a vertex portion 45 (a portion protruding highest in level with respect to the outer circumference of the stent body 20) and then declines gradually from the vertex portion 45 toward the proximal end side. In addition, as shown in Fig. 2 and Figs. 4(a) and 4(b), the portion of the bulging portion 40 along the circumferential direction of the stent body 20 also has a shape which bulges gradually higher in level from one circumferential end toward the vertex portion 45 and then declines gradually from the vertex portion 45 toward the other circumferential end. Therefore, the bulging portion 40 has a shape bulging smoothly as a whole without any corner portion, any edge portion or the like in the circumferential surface of the bulging portion 40.

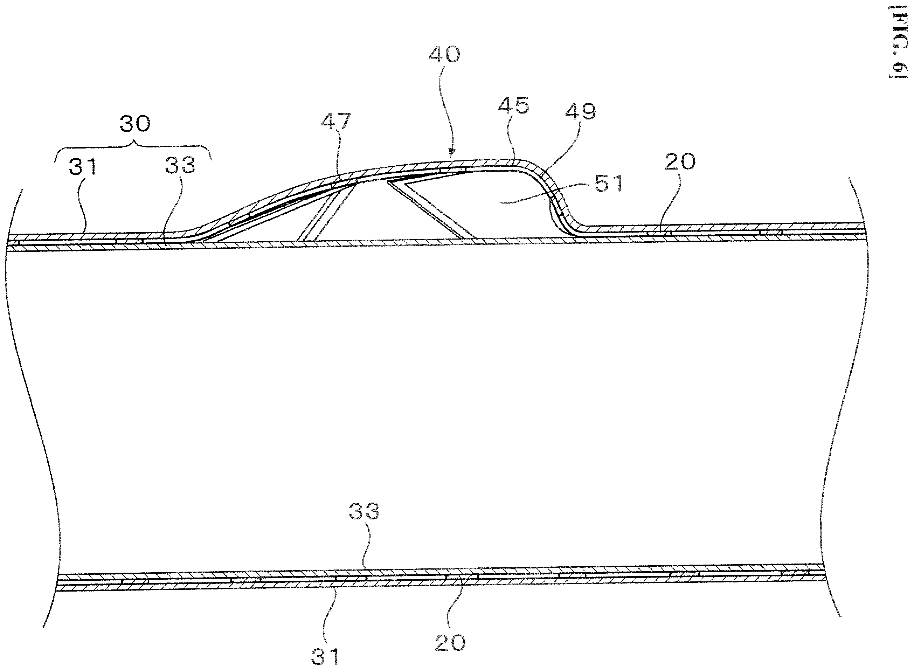

As shown in Fig. 5(b) and Fig. 6, inclination of the bulging portion 40 along the axial direction of the stent body 20 is formed so that a slope corresponding to the direction of taking out the stent 10 with respect to the vertex portion 45, that is, a slope 47 extending from the axially distal end forming the tapered shape to the vertex portion 45 is gentler than an opposite slope to the slope 47, that is, a slope 49 extending from the vertex portion 45 to the axially proximal end.

As shown in Fig. 5(a), the width W of the bulging portion 40 along the circumferential direction of the stent body 20 is formed to be preferably 5% to 60%, more preferably 10% to 40% relative to an outer diameter D of the stent body 20. When the width W of the bulging portion 40 is small than 5% relative to the outer diameter D of the stent body 20, the contact area of the bulging portion 40 with the inner wall of the tubular organ in the circumferential direction is so small that there is a tendency that the stent 10 moves easily. When the width W exceeds 60% relative to the outer diameter D of the stent body 20, the contact area of the bulging portion 40 with the inner wall of the tubular organ is excessively large, so that there is a tendency that it is difficult to take out the stent 10 from the inside of the tubular organ.

As shown in Fig. 5(a), the length L of the bulging portion 40 along the axial direction of the stent body 20 is formed to be preferably larger than the width W of the bulging portion 40. Specifically, the length L of the bulging portion 40 is formed to be preferably 120% to 400%, more preferably 150% to 300% relative to the width W of the bulging portion 40. When the length L of the bulging portion 40 is smaller than 120% relative to the width W, there is a tendency that the stent 10 moves easily inside the tubular organ. When the length L exceeds 400% relative to the width W, there is a tendency that flexibility of the stent 10 decreases.

As shown in Fig. 5(a), an angle of the tapered portion on the axially distal end side of the bulging portion 40, that is, an angle θ between one edge 43 and the other edge 43 on the axially distal end side of the bulging portion 40 is preferably 10° to 50°, more preferably 20° to 45°. When the aforementioned θ is smaller than 10°, it may be difficult to ensure the width of the bulging portion 40. When the aforementioned θ exceeds 50°, there is a tendency that the bulging portion 40 makes resistance to take out the stent 10 from the inside of the tubular organ.

As shown in Fig. 5(b), the height H of the vertex portion 45 of the bulging portion 40 from the outer circumference of the stent body 20 is formed to be preferably 10% to 40%, more preferably 15% to 30% relative to the outer diameter D of the stent body 20. When the height H of the vertex portion 45 of the bulging portion 40 is smaller than 10% relative to the outer diameter D of the stent body 20, there is a tendency that an anchoring effect on the inner wall of the tubular organ decreases. When the height H exceeds 40% relative to the outer diameter D of the stent body 20, there is a possibility that the bulging portion 40 makes close contact with the inner wall of the tubular organ so intensely that it is difficult to take out the stent 10 from the inside of the tubular organ.

As shown in Fig. 6, a space 51 is formed inside each of the bulging portions 40. In the embodiment, the inner cover 33 is disposed on the inner circumference of the stent body 20 so that the spaces 51 are formed between the inner cover 33 and the bulging portions 40.

An example of a method for using the stent 10 having the aforementioned structure will be described below.

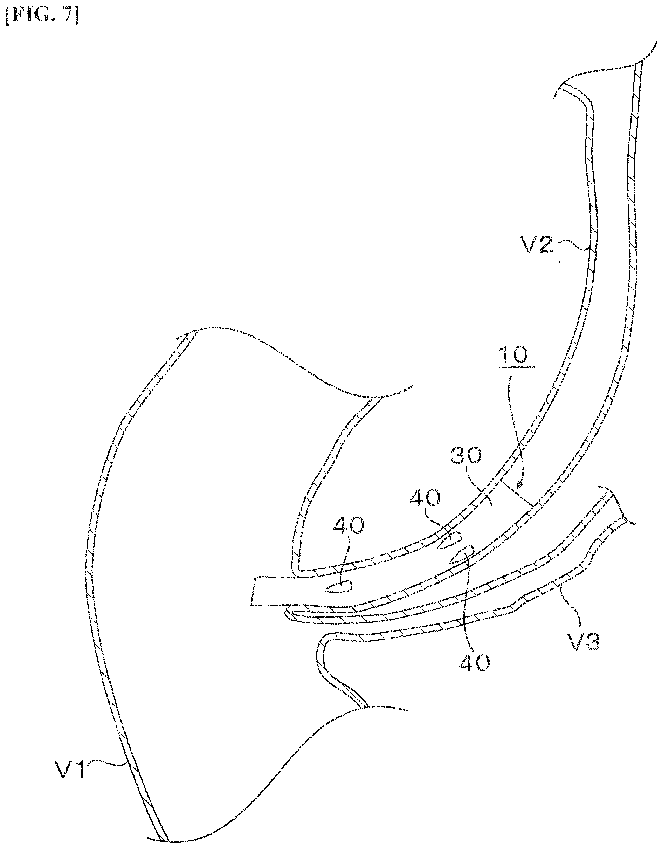

As shown in Fig. 7, the bile duct V2 and the pancreatic duct V3 branch and extend from the duodenum V1. The embodiment will be described in the case where the stent 10 is placed in the bile duct V2. The stent 10 according to the invention may be placed in a tubular organ such as the trachea, the gullet, the large intestine or the blood vessel besides the bile duct V2. However, the tubular organ to which the stent 10 according to the invention can be applied is not particularly limited.

First, the stent 10 whose diameter has been reduced is received in an inner circumference of a distal end portion of a not-shown medical tube such as a catheter or a sheath. On this occasion, the stent 10 is received in the inner circumference of the distal end portion of the medical tube such that the tapered portion of each bulging portion 40 on the axially distal end side faces the proximal end side (operator's hand side) of the medical tube and the expanded diameter portion of the bulging portion 40 on the axially other end side faces the distal end side of the medical tube.

According to a well known method, a not-shown endoscope is operated to pass through the oral cavity, the stomach, etc. and move to the duodenum V1 and a not-shown guide wire is introduced into the bile duct V2 through the lumen of the endoscope so that a distal end portion of the guide wire reaches a position slightly beyond a stenosed affected site of the bile duct V2. Then, the medical tube with the stent 10 received therein is conveyed through the guide wire to thereby bring the distal end portion of the medical tube to the affected site of the bile duct V2. The guide wire is pulled out from the medical tube in this state and a pusher etc. is inserted into the medical tube so as to push the stent 10 out of the distal end of the medical tube through the pusher etc. In this manner, the stent 10 is expanded in diameter, as shown in Fig. 7.

As a result, the stenosed affected site of the bile duct V2 is pushed and widened by the stent 10 and the outer circumference of the stent 10 makes close contact with the inner wall of the bile duct V2 so that the bulging portions 40 go into the inner wall of the bile duct V2. In this manner, when the stent 10 is placed in the bile duct V2 according to the embodiment, the stent 10 makes close contact with the inner wall of the bile duct V2 so that the bulging portions 40 of the stent 10 go into the inner wall of the bile duct V2. Accordingly, displacement of the stent 10 can be suppressed so that the stent 10 can be placed accurately in the affected site of the bile duct V2. The stent 10 is preferably placed with its one end portion slightly protruding from an opening of the bile duct V2 (see Fig. 7).

On this occasion, the stent 10 according to the invention does not have a structure to be placed inside the tubular organ by a biodegradable stent like the double-structure stent in Patent Literature 1. Accordingly, the stent 10 according to the invention can be placed inside the tubular organ stably for a long term without displacement.

According to the embodiment, the bulging portions 40 are disposed in plural places at predetermined intervals in the circumferential direction and the axial direction of the outer circumference of the stent body 20. Accordingly, the bulging portions 40 apply balanced and uniform fixing force. Further, as shown in Fig. 2, Fig. 4(a), and Fig. 7, the bulging portions 40 are disposed so as not to overlap one another when the stent body 20 is viewed from one axially end portion. Accordingly, axial movement of the stent 10 can be prevented more effectively.

In the case where the stent 10 is desired to be taken out from the tubular organ because the lumen of the stent 10 is occluded by a body fluid such as bile or after treatment has been completed, for example, the portion of the stent 10 protruding from the opening of the bile duct V2 (see Fig. 7) or a predetermined place of the stent 10 is gripped by medical snares, medical clamps or the like so that the stent 10 is pulled and extracted from the bile duct V2 and moved to the duodenum V1. Then, the stent 10 is extracted through the catheter etc. Thus, the stent 10 can be taken out from the inside of the body.

On this occasion, each of the bulging portions 40 in the stent 10 has a shape protruding smoothly so as to bulge gradually and then decline gradually. Accordingly, even when the stent 10 is pulled to move while the bulging portions 40 make close contact with the inner wall of the bile duct V2, the inner wall of the bile duct V2 can be prevented from being damaged, so that the stent 10 can be extracted relatively smoothly from the bile duct V2.

According to the embodiment, the outer cover 31 is disposed on the outer circumference of the stent body 20. Accordingly, the stent body 20 can be prevented from making direct and close contact with the inner wall of the bile duct V2 so that the inner wall of the tubular organ can be prevented from biting into meshes of the stent body 20. Thus, the stent 10 can be extracted easily from the tubular organ. Further, the inner cover 33 is disposed also on the inner circumference of the stent body 20. Accordingly, a body fluid such as bile can be prevented from entering into the meshes of the stent body 20 and fluid resistance can be reduced.

Since the spaces 51 are formed inside the bulging portions 40, the bulging portions 40 can be deformed easily when the stent 10 is pulled to be extracted from the inside of the bile duct V2. Thus, the stent 10 can be extracted more smoothly from the bile duct V2.

The inclination of each bulging portion 40 along the axial direction of the stent body 20 is formed so that the inclination of the slope 47 corresponding to the direction of taking out the stent 10 with respect to the vertex portion 45 is gentler than the inclination of the opposite slope 49 to the slope 47 (see Fig. 5(b)). Accordingly, when the stent 10 is extracted from the bile duct V2, the bulging portion 40 is not caught on the inner wall of the bile duct V2 but can be moved smoothly while making close contact with the inner wall. Thus, the stent 10 can be extracted more smoothly from the bile duct V2.

As shown in Fig. 5(a), the bulging portion 40 in the embodiment is formed so that the length L of the bulging portion 40 along the axial direction of the stent body 20 is larger than the width W of the bulging portion 40 along the circumferential direction of the stent body 20. Accordingly, the contact area of the bulging portion 40 with the inner wall of the tubular organ can be kept as large as possible in the axial direction so that the fixing force of the bulging portion 40 can be increased. In addition, when the stent 10 is extracted from the inside of the tubular tube, the stent 10 can be extracted smoothly due to the bulging portion 40 long in the axial direction while preventing damage on the tubular organ.

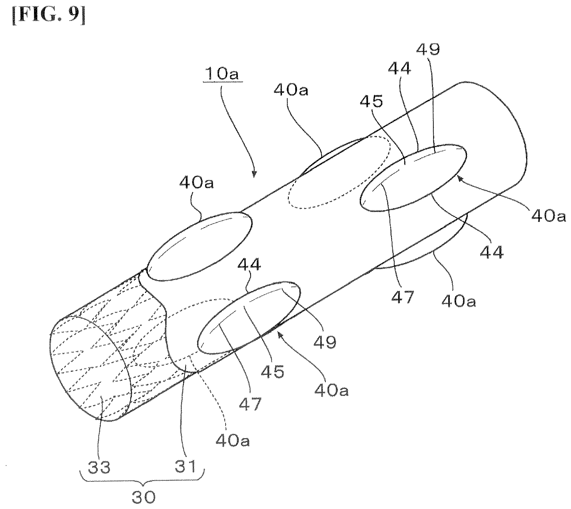

Another embodiment of the stent according to the invention will be described with reference to Fig. 9. Substantially identical parts to those in the previous embodiment are referred to by the same numerals correspondingly so that description thereof will be omitted.

In a stent 10a according to this embodiment, bulging portions 40a have a different shape from those according to the previous embodiment. As shown in Fig. 9, each of the bulging portions 40a in this embodiment is formed so that the length of the bulging portion 40a along the axial direction of the stent body 20 is larger than the width of the bulging portion 40a along the circumferential direction. Thus, the bulging portion 40a has a smoothly-bulged elliptical-like shape as a whole. In the bulging portion 40a, an axially distal end side slope 47 bulging higher in level gradually toward a vertex portion 45 and an axially proximal end side slope 49 declining gradually from the vertex portion 45 draw an arc shape with the same radius and a lower circumferential edge 44 has an elliptical shape.

Three bulging portions 40a are disposed in places closer to one axial end of the stent body 20 at circumferentially equal intervals while three bulging portions 40a are disposed in places closer to the other axial end of the stent body 20 at circumferentially equal intervals so as not to overlap the bulging portions 40a provided in the places closer to the one end. On the other hand, bulging portions 40a may be provided in opposite places in the circumferential direction of the stent body 20, or four bulging portions 40a or more may be provided at equal intervals in the circumferential direction of the stent body 20, in the same manner as in the previous embodiment.

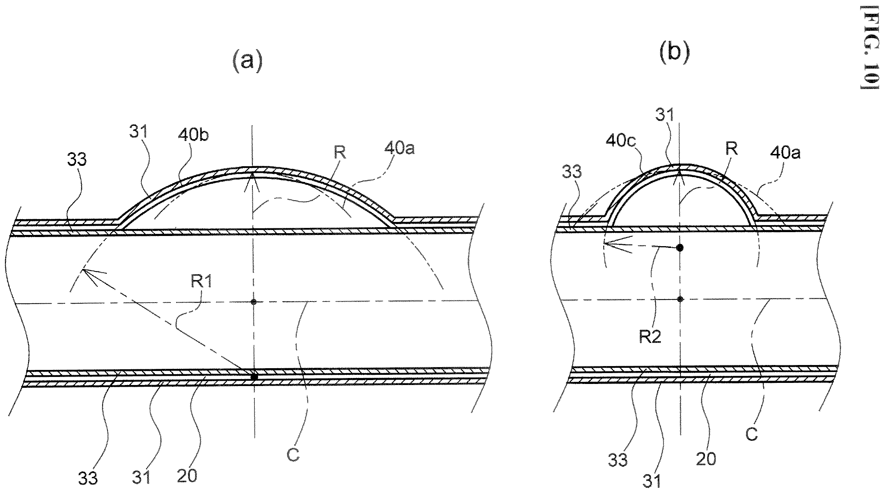

The elliptic-like-shaped bulging portion 40a preferably draws an arc shape in terms of sectional shape along the axial direction of the stent body 20. In this case, the radius of the arc is preferably determined as follows. That is, when a distance between the vertex of the bulging portion 40a of the stent body 20 and the shaft center C of the stent body 20 is designated as radius R, the bulging portion is preferably shaped like an arc having a radius R1 larger than the radius R (bulging portion 40b) as shown in Fig. 10(a), an arc having a radius R2 smaller than the radius R (bulging portion 40c) as shown in Fig. 10(b) or an arc having the radius R. It is more preferred that the bulging portion is shaped like the arc having the radius R1 larger than the radius R.

### Reference Signs List

- 10, 10a: stent
- 20: stent body
- 30: cover member
- 40, 40a, 40b, 40c: bulging portion
- 47, 49: slope
- 51: space

## Claims

1. A stent (10) which is placed in a tubular organ, including:
a metal stent body (20);
and
bulging portions (40, 40a, 40b, 40c) which are provided in the part of the outer circumference of the stent body (20) covered with a resin cover member (30) and which make close contact with an inner wall of the tubular organ so as to restrain the stent (10) from moving,
wherein each of the bulging portions (40, 40a, 40b, 40c) has a gradually-bulged shape which extends with a predetermined width in a circumferential direction and with a predetermined length in an axial direction so as to bulge gradually and then decline gradually, and
wherein a space (51) is formed inside each of the bulging portions (40, 40a, 40b, 40c);
**characterized in that** it further comprises
a resin cover member (30) which covers at least a part of an outer circumference of the stent body (20), whereby
the resin cover member (30) includes an outer cover (31) and an inner cover (33), and
the space (51) is formed between the inner cover (33) and the bulging portion (40).

2. The stent (10) of Claim 1,
wherein the bulging portions (40, 40a, 40b, 40c) are disposed in plural places at predetermined intervals along the circumferential direction of the stent body (20) and in plural places at predetermined intervals also along the axial direction of the stent body (20) so as not to overlap one another in axial view from an end face of the stent body (20).

3. The stent (10) of Claim 1 or 2,
wherein each of the bulging portions (40, 40a, 40b, 40c) is formed so that the length of the bulging portion (40, 40a, 40b, 40c) along the axial direction of the stent body (20) is larger than the width of the bulging portion (40, 40a, 40b, 40c) along the circumferential direction of the stent body (20).

4. The stent (10) of any one of Claims 1 to 3,
wherein inclination of each of the bulging portions (40, 40a, 40b, 40c) along the axial direction of the stent body (20) is formed so that inclination of a slope (47) corresponding to a direction of taking out the stent with respect to a vertex portion is gentler than inclination of an opposite slope (49).

## Patentansprüche

1. Stent (10), der in ein röhrenförmiges Organ eingesetzt wird, wobei er enthält:
einen Stent-Körper (20) aus Metall;
sowie
Wölbungsabschnitte (40, 40a, 40b, 40c), die sich in dem Teil des Außenumfangs des Stent-Körpers (20) befinden, der mit einem Kunststoff-Abdeckungselement (30) abgedeckt ist und der in engem Kontakt mit einer Innenwand des röhrenförmigen Organs ist, um so den Stent (10) an Bewegung zu hindern,
wobei jeder der Wölbungsabschnitte (40, 40a, 40b, 40c) eine sich allmählich wölbende Form hat, die sich mit einer vorgegebenen Breite in einer Umfangsrichtung sowie mit einer vorgegebenen Länge in einer axialen Richtung erstreckt und sich allmählich wölbt und anschließend allmählich abfällt, und
wobei ein Raum (51) im Inneren jedes der Wölbungsabschnitte (40, 40a, 40b, 40c) ausgebildet ist;
**dadurch gekennzeichnet, dass** er des Weiteren umfasst:
ein Kunststoff-Abdeckungselement (30), das wenigstens einen Teil eines Außenumfangs des Stent-Körpers (20) abdeckt, wobei
das Kunststoff-Abdeckungselement (30) eine äußere Abdeckung (31) sowie eine innere Abdeckung (33) einschließt, und
der Raum (51) zwischen der inneren Abdeckung (33) und dem Wölbungsabschnitt (40) ausgebildet ist.

2. Stent (10) nach Anspruch 1,
wobei die Wölbungsabschnitte (40, 40a, 40b, 40c) an mehreren Positionen in vorgegebenen Abständen in der Umfangsrichtung des Stent-Körpers (20) und an mehreren Positionen in vorgegebenen Abständen ebenfalls in der axialen Richtung des Stent-Körpers (20) so angeordnet sind, dass sie einander in einer axialen Ansicht von einer Endfläche des Stent-Körpers (20) her nicht überlappen.

3. Stent (10) nach Anspruch 1 oder 2,
wobei jeder der Wölbungsabschnitte (40, 40a, 40b, 40c) so ausgebildet ist, dass die Länge des Wölbungsabschnitts (40, 40a, 40b, 40c) in der axialen Richtung des Stent-Körpers (20) größer ist als die Breite des Wölbungsabschnitts (40, 40a, 40b, 40c) in der Umfangsrichtung des Stent-Körpers (20).

4. Stent (10) nach einem der Ansprüche 1 bis 3,
wobei die Neigung jedes der Wölbungsabschnitte (40, 40a, 40b, 40c) in der axialen Richtung des Stent-Körpers (20) so ausgebildet ist, dass die Neigung einer Abschrägung (47), die einer Richtung entspricht, in der der Stent entnommen wird, in Bezug auf einen Scheitelabschnitt kleiner ist als die Neigung einer gegenüberliegenden Abschrägung (49).

## Revendications

1. Stent (10) qui est placé dans un organe tubulaire, incluant:
un corps de stent métallique (20); et
des parties en saillie (40, 40a, 40b, 40c) qui sont agencées dans la partie de la circonférence externe du corps de stent (20) couverte par un membre de couverture en résine (30) et qui sont en contact étroit avec une paroi interne de l'organe tubulaire de manière à empêcher le stent (10) de se déplacer,
où chacune des parties en saillie (40, 40a, 40b, 40c) a une forme progressivement en saillie qui s'étend avec une largeur prédéterminée dans une direction de la circonférence et avec une longueur prédéterminée dans une direction axiale de manière à faire saillie progressivement puis à décliner progressivement,
et
où un espace (51) est formé à l'intérieur de chacune des parties en saillie (40, 40a, 40b, 40c);
**caractérisé en ce qu'**il comprend en outre
un membre de couverture en résine (30) qui couvre au moins une partie d'une circonférence externe du corps de stent (20), où le membre de couverture en résine (30) inclut une couverture externe (31) et une couverture interne (33), et
l'espace (51) est formé entre la couverture interne (33) et la partie en saillie (40).

2. Stent (10) selon la revendication 1,
où les parties en saillie (40, 40a, 40b, 40c) sont disposées en plusieurs emplacements à des intervalles prédéterminés dans la direction de la circonférence du corps de stent (20) et en plusieurs emplacements à des intervalles prédéterminés dans la direction axiale du corps de stent (20) de manière à ne pas se chevaucher les unes les autres en vue axiale depuis une face d'extrémité du corps de stent (20).

3. Stent (10) selon la revendication 1 ou 2,
où chacune des parties en saillie (40, 40a, 40b, 40c) est formée de sorte que la longueur de la partie en saillie (40, 40a, 40b, 40c) dans la direction axiale du corps de stent (20) est plus grande que la largeur de la partie en saillie (40, 40a, 40b, 40c) dans la direction de la circonférence du corps de stent (20).

4. Stent (10) selon l'une quelconque des revendications 1 à 3,
où l'inclinaison de chacune des parties en saillie (40, 40a, 40b, 40c) dans la direction axiale du corps de stent (20) est formée de telle sorte que l'inclinaison d'une pente (47) correspondant à une direction de retrait du stent par rapport à une partie formant sommet est plus douce que l'inclinaison d'une pente opposée (49).
